Europäisches Patentamt

European Patent Office

Office européen des brevets

(19)

(11) Veröffentlichungsnummer: **0 142 016**
**A2**

(12) **EUROPÄISCHE PATENTANMELDUNG**

(21) Anmeldenummer: **84112072.8**

(22) Anmeldetag: **09.10.84**

(51) Int. Cl.⁴: **C 07 C 19/045**
**C 07 C 17/02**

(30) Priorität: **10.11.83 DE 3340624**

(43) Veröffentlichungstag der Anmeldung:
**22.05.85 Patentblatt 85/21**

(84) Benannte Vertragsstaaten:
**BE DE FR GB IT NL SE**

(71) Anmelder: **DYNAMIT NOBEL AKTIENGESELLSCHAFT**
**Postfach 1261**
**D-5210 Troisdorf, Bez. Köln(DE)**

(72) Erfinder: **Leuck, Hans**
**Robert-Koch-Strasse 4**
**D-5210 Troisdorf 15(DE)**

(72) Erfinder: **Westermann, Hans-Jörg**
**Hitzbroicher Weg 18**
**D-5210 Troisdorf 15(DE)**

(54) Verfahren zur Herstellung von 1,2-Dichlorethan aus Ethylen und Chlorgas.

(57) Bei diesem Verfahren zur Herstellung von 1,2-Dichlorethan aus Ethylen und Chlorgas in einem etwa äquimolaren Mengenverhältnis bei Reaktionsdrücken zwischen 2 und 20 bar und Siedetemperaturen des gebildeten Ethylendichlorids zwischen 105 und 225°C in Gegenwart von als Lewissäuren wirkenden Katalysatoren in einem Schlaufenreaktor werden die bei der Verdampfungskühlung anfallenden katalysatorfreien Brüden von Ethylendichlorid unter nachfolgender Kondensation und Kühlung sowie getrennt flüssiges katalysatorhaltiges Ethylendichlorid abgezogen.

Zwecks Umgehung einer Chlorgaskompression und Durchführung des Verfahrens so, daß die Bildung höher chlorierter Produkte sowie von chlorhaltigen Destillationsrückständen weitgehend unterdrückt wird, wird das Verfahren so durchgeführt, daß

a) in einen kondensierten und gekühlten Kreislaufstrom von Ethylendichlorid das gesamte eingesetzte gasförmige Chlor, das eine Reinheit von etwa 90 bis 100 Vol.-% aufweist, eingeleitet,

b) der erhaltene chlorhaltige Ethylendichlorid-Strom auf Reaktionsdruck gebracht und anschließend aus dem Reaktor abgezogenes katalysatorhaltiges Ethylendichlorid zugemischt,

c) der chlorhaltige und katalysatorhaltige Ethylendichloridstrom unter Abkühlung des katalysatorfreien Ethylendichloridstromes, von dem dann das Endprodukt Ethylendichlorid als Teilstrom abgenommen wird, hochgeheizt,

d) danach in den Abwärtsstrom des Reaktors unter nachfolgender Zugabe eines Ethylen-Teilstromes eingespeist und

e) der Abwärtsstrom beim Kontakt mit einer am Boden des Reaktors eingeleiteten Ethylenhaupteinleitungsmenge bei einer Massengeschwindigkeit zwischen 30 und 200 kg/sec. m² dieser Ethylenmenge in einen eine hochdisperse Gas-Flüssigkeitsphase bildenden Aufwärtsstrom umgelenkt wird.

EP 0 142 016 A2

Troisdorf, den 7.11.1983
OZ 83066 (4291) Dr.Li/br.

DYNAMIT NOBEL AKTIENGESELLSCHAFT

5210 Troisdorf

## Verfahren zur Herstellung von 1,2-Dichlorethan aus Ethylen und Chlorgas

Die Erfindung betrifft ein Verfahren der im Oberbegriff von Patentanspruch 1 angegebenen Art.

Es ist bekannt, 1,2-Dichlorethan (nachfolgend auch Ethylendichlorid genannt) dadurch herzustellen, daß Ethylen mit Chlor in flüssigem Ethylendichlorid in Gegenwart von Lewis-Säurekatalysatoren zur Reaktion gebracht wird. Die bekannten großtechnischen Verfahren arbeiten dabei kontinuierlich, mit Normaldruck oder leicht erhöhtem Druck, unterhalb oder bei Siedetemperatur des Ethylendichlorids. Man ist hierbei bestrebt, hohe Stoff- und Raumzeitausbeuten bei möglichst quantitativem Ethylenumsatz zu erzielen und bei konstanter Temperatur im Reaktor die beträchtliche Reaktionswärme abzuführen (vgl. DE-PS 24 27 045). Um diese Forderungen zu erfüllen, werden verschiedene Wege beschritten.

So wird die Reaktionswärme über ein innerhalb des Reaktors installiertes Kühlsystem abgeführt oder die Durchführung

- 2 -

der Chlorierungsreaktion einerseits und die Ableitung der Reaktionswärme andererseits erfolgt in getrennten Apparaturen, wobei dem gebildeten Produkt durch Temperatursenkung in außerhalb des Reaktors angeordneten Kühlern Wärme entzogen wird. Beide Kühlsysteme werden allmählich durch Ablagerungen von Komplexverbindungen und schwerlöslichen Produkten in ihrer Wirksamkeit stark eingeschränkt, so daß zur Aufrechterhaltung der Leistungsfähigkeit der Anlagen in periodischen Abständen aufwendige Reinigungsarbeiten notwendig sind.

Die DE-OS 29 35 885 beschreibt eine Kombination zweckmäßiger chemischer und verfahrenstechnischer Maßnahmen, bei der u.a. die Hauptreaktion der Addition von Chlor an Ethylen bei Drücken von 0,3 bis 1,3 bar und Temperaturen von 50 bis 90 °C durchgeführt, mindestens die Hälfte des Chlors gelöst im gekühlten Reaktionsprodukt zum Einsatz kommt und das restliche Chlor gasförmig und/oder flüssig zugegeben wird.

Neuere Verfahren arbeiten bei Temperaturen von über 100 °C, um ein ausreichendes Temperaturniveau für die Nutzung der Reaktionswärme von etwa 1 t Dampf/t Ethylendichlorid zu haben. Die Wärmeabfuhr erfolgt dabei ebenfalls durch Direktkühlung des Reaktorinhaltes oder durch Verdampfungskühlung von Ethylendichlorid, wobei die Regenerierung der Reaktionswärme in der Regel dadurch geschieht, daß mit Kondensat unter Dampfgewinnung gekühlt bzw. kondensiert wird oder andere Verfahrenseinheiten direkt mit den anfallenden Dämpfen von Ethylendichlorid beheizt werden.

Weitere Unterschiede liegen in der Einspeisung der Ausgangsprodukte Ethylen und Chlor in das Reaktionssystem sowie bei der Benutzung von Luftsauerstoff als Prozeßhilfsmittel. Bei einigen Verfahren werden die Reaktionsgase direkt in den Reaktor eingeleitet, bei anderen ganz oder teilweise in außerhalb des Reaktors befindlichen Misch- und Lösungseinrichtungen mit Ethylendichlorid in Kontakt und anschließend zur Reaktion gebracht.

Hierbei wirft die Ethylendichlorid-Herstellung bei höherer Temperatur und damit erhöhtem Druck durch das meist direkt aus einer Chlor-Alkali-Elektrolyse kommende Zellenchlor und die darin enthaltenen gasförmigen Verunreinigungen von bis zu 10 % ($H_2$, $N_2$, $O_2$ und $CO_2$) besondere Probleme auf. So muß das Chlorgas je nach Reaktionstemperatur auf einen Druck zwischen 5 und 20 bar komprimiert werden, was technisch aufwendig ist und viel Antriebsenergie benötigt.

Darüber hinaus enthalten die das Reaktionssystem verlassenden Abgase nach der Abtrennung des Ethylendichlorids geringe Mengen an Chlorwasserstoff und Chlor, die meist mit Natronlauge neutralisiert werden. Das danach vorliegende Gemisch aus restlicher Natronlauge, Kochsalz, Natriumhypochlorit und Natriumcarbonaten in Wasser muß entsorgt werden. Die gleiche Notwendigkeit besteht bei dem Abzug von flüssigem Ethylendichlorid aus dem Reaktor und der dabei erforderlichen Neutralisation des Chlorwasserstoffs; neben den genannten Abfallstoffen fällt hierbei noch eine beträchtliche Menge an Eisenhydroxydschlamm bei der Verwendung von Eisen(III)-Chlorid /als Katalysator/ in der Dekantiereinrichtung zur Trennung von organischer und wäßriger Phase an. Bei den erforderlichen periodischen Reinigungsarbeiten und im sonstigen Verfahrensablauf treten Stoffverluste auf, die entsprechenden Katalysatorverluste müssen laufend ausgeglichen werden.

Der Erfindung liegt die Aufgabe zugrunde, bei einem Verfahren der im Oberbegriff des Patentanspruches 1 bezeichneten Art eine Chlorgaskompression zu umgehen und das Verfahren so durchzuführen, daß die Bildung höher chlorierter Produkte sowie von chlorhaltigen Destillationsrückständen weitgehend unterdrückt werden.

Diese Aufgabe wird durch die kennzeichnenden Merkmale des Anspruches 1 gelöst.Vorteilhafte Ausgestaltungen der Erfindung sind in den Unteransprüchen angegeben.

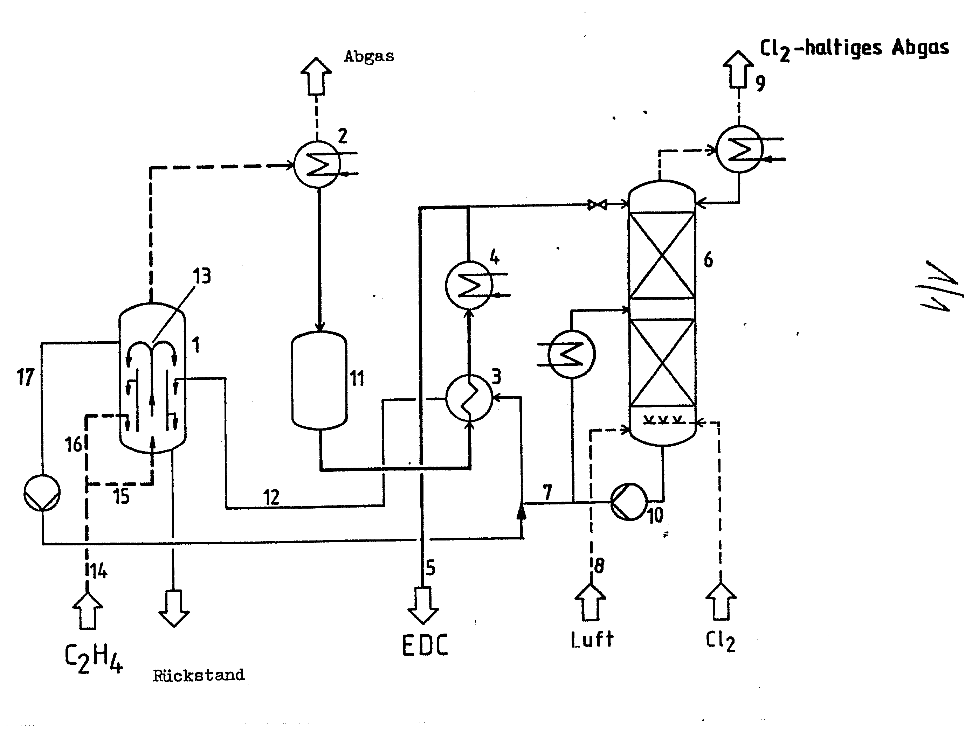

Die erfindungsgemäßen Merkmale werden nachfolgend und unter Bezugnahme auf die Figur der Zeichnung erläutert. Die Figur zeigt ein Schema, in dem das erfindungsgemäße Verfahren beispielhaft ausgeführt ist und soll nicht einschränkend wirken.

Die im Reaktor 1 anfallenden Brüden werden zur Dampfgewinnung im Kondensator 2 kondensiert und unter Zwischenschaltung eines Vorlagebehälters 11 in weiteren Wärmeaustauschern 3 und 4 auf 30 bis 40 $^{\circ}$C abgekühlt. Von dem Produktstrom von rohem Ethylendichlorid wird über Leitung 5 die Produktmenge an rohem Ethylendichlorid zur weiteren Aufarbeitung zum Endprodukt abgezweigt. Die Differenzmenge entspricht etwa dem 5- bis 8fachen der in Leitung 5 abgezweigten Menge und wird zum Lösen des Einsatzchlors in Absorber 6 eingesetzt. Dementsprechend weist der den Absorber verlassende Stoffstrom 7 eine Chlorkonzentration zwischen etwa 8 und 13 Gew.-% auf. Bei einer Reaktortemperatur von 105 $^{\circ}$C soll der Chlorvordruck entsprechend dem Lösungsverhalten von Chlor in Ethylendichlorid bei mindestens 2,5 bar liegen, während bei einer Reaktortemperatur von 225 $^{\circ}$C der Chlorvordruck nur noch etwa 1,2 bar zu betragen braucht. Dabei

- 5 -

werden auch die - bei Verwendung von Zellenchlor aus der Chlor-Alkali-Elektrolyse - im Chlor enthaltenen gasförmigen Komponenten $CO_2$ und $O_2$ annähernd quantitativ, $H_2$ und $N_2$ kaum gelöst.

Die weitgehende Absorption des $CO_2$ erhöht die Gesamt-wirtschaftlichkeit des Verfahrens insofern, als ein $CO_2$-freies chlorhaltiges Abgas, Stoffstrom 9, hinter dem Absorber nach weitgehender Entfernung von Ethylendichlorid der Neutralisation mit wäßriger Natronlauge zugeführt wird. Die entstehende Lauge kann als Bleichlauge weiterverwendet werden.

Es ist bekannt, daß elementarer Sauerstoff einen positiven Einfluß auf die Selektivität der Additionsreaktion von elementarem Chlor an Ethylen ausübt. Deshalb besteht eine bevorzugte Ausgestaltung des Verfahrens darin, den Sauerstoff zusätzlich in Form von Luft, Stoffstrom 8, in das Chlor-Absorptionssystem einzuleiten, statt wie üblich direkt in den Reaktor.

Nach erfolgter Chlorabsorption bei niedrigem Druck wird die erhaltene Chlor-Ethylendichlorid-Lösung mit Pumpe 10 auf den gewünschten erhöhten Reaktordruck gebracht, mit aus dem Reaktor abgezogenem katalysatorhaltigem Ethylendichlorid, Stoffstrom 17, versetzt und im Gegenstrom zu den kondensierten Brüden in Wärmeaustauscher 3 auf eine Temperatur angehoben, die um 20 $^\circ$C oder weniger unter-halb der Reaktionstemperatur liegt.

- 6 -

Dieses Aufheizen ist notwendig, damit im Reaktor 1 genügend Ethylendichlorid verdampft und nach der Brüdenkondensation in Kondensator 2 hinter dem Reaktor genügend Ethylendichlorid im Vorlagebehälter 11 zum Lösen des Einsatzchlors vorliegt. Durch diese Temperaturerhöhung verdampft entsprechend dem Lösungsgleichgewicht ein Teil des absorbierten Chlors, wobei sich in dem Wärmeaustauscher 3 eine Zweiphasen-Strömung ausbildet. Da aber Versuche gezeigt haben, daß die Selektivität der Reaktion nachteilig beeinflußt wird, wenn Chlor-Ethylen-Gasphasenreaktionen auftreten können, die zu unerwünschten, insbesondere höher chlorierten Nebenprodukten führen, ist es bei dem erfindungsgemäßen Verfahren wichtig, daß dieser verdampfte Chloranteil vor dem Zusammenbringen mit dem eingesetzten Ethylen wieder in Lösung vorliegt. Dies wird durch Anwendung des Schlaufenreaktor-Prinzips in 1 erreicht, wodurch dem chlorhaltigen Zweiphasen-Einsatzstrom 12 ein großer, chlorfreier, katalysatorhaltiger Ethylendichlorid-Umlaufstrom 13 überlagert wird.

Das Mengenverhältnis der beiden Ströme 12 und 13 wird dabei so gewählt, daß die Chlorkonzentration im Summenstrom unter Berücksichtigung von Reaktionstemperatur und Senkung des Chlorpartialdrucks durch verdampfendes Ethylendichlorid höchstens so hoch ist, wie der Löslichkeitskonzentration des Chlors in Ethylenchlorid entspricht.

- 7 -

Es ist ferner bekannt, daß auch die Katalysatorkonzentration sowohl den Umsatz als auch die Selektivität der Ethylendichlorid-Bildung beeinflußt. Untersuchungen haben gezeigt, daß der Umsatz bis zu einer bestimmten Katalysatorkonzentration ansteigt, um dann in etwa konstant zu bleiben. Überraschenderweise sinkt jedoch die Selektivität und damit die Stoffausbeute bei höheren Katalysatorkonzentrationen. In einer vorteilhaften Ausgestaltung liegt die Katalysatorkonzentration für den angewandten Eisen(III)-Chlorid-Katalysator zwischen 300 und 1 000 Gew.-ppm.

Die Erfindung bietet den besonderen Vorteil, daß die geforderte Katalysatorkonzentration im Bereich der Hauptreaktionszone durch den Ethylenseitenstrom, durch die Größe des Ethylendichlorid-Kreislaufstromes sowie durch die Menge des rückgeführten katalysatorhaltigen Stroms 17 genau eingestellt werden kann.

Die Art der Zusammenführung des Ethylens mit dem chlor- und katalysatorhaltigen Umlauf-Ethylendichlorid ist charakteristisch für das vorliegende Verfahren. Untersuchungen (Balasubramanian, S.N. et al, IGC Fund, $\underline{5}$ (1966) 184) und eigene Versuche zeigten, daß die Reaktion zwischen Ethylen und Chlor augenblicklich abläuft und daß das Gleichgewicht weit auf der Seite des 1,2-Dichlorethans liegt; die Reaktion läuft praktisch vollständig ab, wobei der Stoffaustausch die geschwindigkeitsbestimmende Größe ist. In dem vorliegenden Verfahren wird deshalb dafür gesorgt, daß mit Hilfe des gasförmigen Reaktionspartners Ethylen, Strom 14, beim Kontakt mit der Flüssigkeitsphase in dem danach im Reaktor 1 vorliegenden heterogenen Gas-Flüssigkeitssystem hydrodynamische Verhältnisse herrschen, die den Stoffübergang zwischen den Phasen fördern. Dies kann dadurch erreicht werden, daß der Hauptteil des als Stoffstrom 14 eingesetzten Ethylens als Stoffstrom 15 über eine oder mehrere Düsen so in den Reaktor 1 mit Massengeschwindigkeiten zwischen 30 und 200 $kg/m^2$. sec eingeleitet wird, daß beim Kontakt mit dem chlorhaltigen und katalysatorhaltigen Abwärtsstrom dieser in den Aufwärtsstrom umgelenkt wird. Damit wird ein hochdisperser Zustand des reagierenden Gas-Flüssigkeitsgemisches bewirkt und der andere Teil des eingesetzten Ethylens als Stoffstrom 16 im Reaktorringraum zur Steuerung der Flüssigkeits-Umlaufzahl benutzt.

Zur weiteren Beschreibung des vorliegenden Verfahrens werden die nachfolgenden Beispiele 1 bis 3 angegeben:

Beispiel 1

In einem Doppelmantel-Reaktor aus Stahlmaterial werden ca. 4 l Reinst-Ethylendichlorid (nachfolgend EDC) mit 1 000 Gew.-ppm Fe-III-Chlorid vorgelegt und auf Reaktionstemperatur hochgeheizt.

In einer speziell gestalteten Düse wird zuerst katalysatorfreies mit katalysatorhaltigem EDC und Reinst-Chlor gemischt, ehe diese Lösung mit Ethylen, das mit einer Massengeschwindigkeit von ca. 35 $kg/m^2$. sec eingedüst wird, zur Reaktion gebracht wird.

Die bei der Reaktion entstehenden Brüden werden kondensiert und in den unteren Teil des Reaktors - nach Abzug der erzeugten Produktmenge - zurückgegeben.

- 9 -

Durch den Mischeffekt der Düse ergeben sich im unteren
Teil des Reaktors Katalysatorkonzentrationen von ca.
50 bis 70 % der mittleren Konzentration von 1 000 ppm.
Eingesetzt wurden 0,61 kg/h $C_2H_4$ und 1,6 kg/h $Cl_2$, was
einem $Cl_2$-Überschuß von ca. 3,5 % entspricht. Die Reaktionstemperatur lag bei 135 °C. Nach 5 h Versuchsdauer
wurde die Zufuhr der Gase abgestellt und der Reaktorinhalt über den Doppelmantel rasch abgekühlt.

Die Auswertung ergab folgende Ergebnisse:

          Umsatz an $C_2H_4$     99,9 %
          davon zu EDC       90,2 %;

das ergibt eine Ausbeute von 90,1 % der Theorie.

Beispiel 2

Versuchsdurchführung und Temperatur wie im Beispiel 1.
Die mittlere Katalysatorkonzentration wurde jedoch auf
200 ppm gesenkt.

Ergebnis:

          Umsatz an $C_2H_4$     96,1 %
          davon zu EDC       90   %;

das ergibt eine Ausbeute von ca. 86,5 % der Theorie.

Beispiel 3 (Vergleichsbeispiel

In einem 2 l-Reaktor aus C-Stahl mit Mantel-Kühlung, Rühreinrichtung, Gaseinleit- und Ablaßsystem und Druck-meßeinrichtung wurden 0,8 l EDC mit 1 000 ppm Fe-III-Chlorid vorgelegt und bei 120 $^{\circ}$C gut gerührt. Durch Gasleitungen im unteren Teil des Reaktors wurden $C_2H_4$ und $Cl_2$ in äquimolaren Mengen eingeleitet, bis sich ein Systemdruck von ca. 10 bar, hervorgerufen durch ungelöste Gase, einstellt. Durch das Abreagieren von $C_2H_4$ nach ca. 15 min stellte sich wieder der zu 120 $^{\circ}$C gehö-rende Druck ein. Dieser Vorgang wurde ca. 20mal wiederholt. Bedingt durch die Versuchsdurchführung lag der Umsatz bei nahezu 100 %. Die Ausbeute lag bei ca. 57 % (als Mittel mehrerer Versuche).

—1— Troisdorf, den 7.11.1983
OZ 83066 (4291) Dr.Li/br.

1 <u>Patentansprüche</u>

1. Verfahren zur Herstellung von 1,2-Dichlorethan aus
   Ethylen und Chlorgas in einem etwa äquimolaren Mengenverhältnis bei Reaktionsdrücken zwischen 2 und 20 bar
   und Siedetemperaturen des gebildeten Ethylendichlorids
   zwischen 105 und 225 $^{\circ}$C in Gegenwart von als Lewissäuren wirkenden Katalysatoren in einem Schlaufenreaktor unter Abzug mit nachfolgender Kondensation und
   Kühlung von bei der Verdampfungskühlung anfallenden
   katalysatorfreien Brüden von Ethylendichlorid sowie
   von flüssigem katalysatorhaltigem Ethylendichlorid,
   dadurch gekennzeichnet, daß

   a) in einen kondensierten und gekühlten Kreislaufstrom
      von Ethylendichlorid das gesamte eingesetzte gasförmige Chlor, das eine Reinheit von etwa 90 bis
      100 Vol.-% aufweist, eingeleitet,

   b) der erhaltene chlorhaltige Ethylendichlorid-Strom
      auf Reaktionsdruck gebracht und anschließend aus
      dem Reaktor abgezogenes katalysatorhaltiges Ethylendichlorid zugemischt,

   c) der chlorhaltige und katalysatorhaltige Ethylendichloridstrom unter Abkühlung des katalysatorfreien
      Ethylendichloridstromes, von dem dann das Endprodukt
      Ethylendichlorid als Teilstrom abgenommen wird,
      hochgeheizt,

   d) danach in den Abwärtsstrom des Reaktors unter nachfolgender Zugabe eines Ethylen-Teilstromes einge-
      speist und

- 2 -

e) der Abwärtsstrom beim Kontakt mit einer am Boden des Reaktors eingeleiteten Ethylenhaupteinleitungsmenge bei einer Massengeschwindigkeit zwischen 30 und 200 kg/sec. $m^2$ dieser Ethylenmenge in einen eine hochdisperse Gas-Flüssigkeitsphase bildenden Aufwärtsstrom umgelenkt wird.

2. Verfahren nach Anspruch 1, dadurch gekennzeichnet, daß zusätzlich Luftsauerstoff in die Chlorabsorptionsstufe eingeleitet wird.

3. Verfahren nach Anspruch 1, dadurch gekennzeichnet, daß das gesamte eingesetzte gasförmige Chlor in den kondensierten und gekühlten Kreislaufstrom von Ethylendichlorid bei einem Chlorvordruck von etwa 1,2 bis mindestens 2,5 bar eingeleitet wird.

4. Verfahren nach Anspruch 1, dadurch gekennzeichnet, daß der chlorhaltige und katalysatorhaltige Ethylendichloridstrom auf eine Temperatur, die um 20 $^\circ$C oder weniger unterhalb der Reaktionstemperatur liegt, hochgeheizt wird.

5. Verfahren nach Anspruch 1, dadurch gekennzeichnet, daß die Umlaufzahl im Schlaufenreaktor zur Einstellung der optimalen Katalysator- und Chlorkonzentration durch Zugabe eines Ethylenteilstromes in den Abwärtsstrom des Schlaufenreaktors gesteuert wird.

6. Verfahren nach Anspruch 1, dadurch gekennzeichnet, daß in dem Aufwärtsstrom im Reaktor eine Katalysatorkonzentration von 300 bis 1 000 Gew.-ppm eingehalten wird.

Abgas

Cl$_2$-haltiges Abgas

13

1

17

16

15

14

C$_2$H$_4$

Rückstand

12

11

2

4

3

5

EDC

7

8

Luft

10

Cl$_2$

6

9

01 42016